# EUROPEAN PATENT SPECIFICATION

(11) **EP 2 119 461 B1**
(45) Date of publication and mention of the grant of the patent: **14.12.2011**
(21) Application number: 09154425.4
(22) Date of filing: 05.03.2009
(51) Int. Cl.: A61M 1/00, A61M 1/02, A61M 1/36

(54) **Postoperative autotransfusion device with improved efficiency**
Vorrichtung zur postoperativen Autotransfusion mit verbesserter Effizienz
Dispositif d'autotransfusion postopératoire avec efficacité améliorée

(30) Priority: 13.05.2008 IT MI20080858
(43) Date of publication of application: 18.11.2009
(73) Proprietor: Eurosets S.r.l., 41036 Medolla (MO) (IT)
(72) Inventor: Tassi, Carlo Alberto, 41038 SAN FELICE SUL PANARO MO (IT); Fontanili, Paolo, 42015 CORREGGIO RE (IT)
(74) Representative: Zoli, Filippo

(56) References cited:
- EP-A- 0 384 585
- US-A- 3 911 918
- US-A- 4 663 032
- US-A- 5 282 982
- US-A- 5 407 425

## Description

The present invention relates to a device for postoperative autotransfusion. More particularly, the invention relates to a device for postoperative autotransfusion that simultaneously allows drainage of a wound.

As is known, in the case of surgical procedures, a postoperative autotransfusion is usually performed after the procedure and consists in collecting blood by way of the drainages of the surgical wound and in infusing it after filtration.

Usually, autotransfusion is performed by means of a device that comprises a collection tank with a connection to the drainages of the surgical wound; the collection tank comprises a supernatant separator partition that allows to separate concentrated erythrocytes from supernatant and block the transfer from the collection tank to a reinfusion container, generally constituted by a bag, from which the blood is reinfused to the patient after passing through a filter.

Usually, the blood collected in the collection tank is stratified by way of the different specific gravity of blood components, undergoing sedimentation over time.

Stratification provides for a first layer of concentrated erythrocytes, i.e., red cells, a layer of white cells, a layer of platelets and a layer of supernatant (plasma fraction plus any contaminating lipid fraction).

It must be possible to perform reinfusion so as to reinfuse only the blood components that can be useful and not harmful for the patient, thus eliminating the supernatant composed of exuded plasma and any contaminating lipid fraction.

This operation is usually performed by using a supernatant separator partition of constant height, which allows to block automatically within the collection tank at all times a constant volume of supernatant and not a variable volume as would be required.

Document US 5 282 982 discloses an apparatus for aspirating a mixture of shed blood and for recovering packed red cells therefrom, that has the features of the preamble of claim 1. The aim of the present invention is to provide a postoperative autotransfusion device that allows to discriminate in an automated manner concentrated erythrocytes from supernatant thereby blocking the transfer from the collection tank to the reinfusion container at the end of the passage of the concentrated erythrocytes.

The expression "reinfusion container" is understood to reference, in addition to any containment object, also the patient himself.

Within this aim, an object of the present invention is to provide a postoperative autotransfusion device in which the supernatant separator partition can be moved in order to block automatically a variable quantity of supernatant within the collection tank.

Another object of the present invention is to provide a postoperative autotransfusion device in which the movement of the separator partition can occur manually as well as in an automated manner.

Another object of the present invention is to provide a postoperative autotransfusion device in which manual movement can occur both on the basis of visual control, by the operator, of the stratification within the collection tank and on the basis of the appropriately received information.

Another object of the present invention is to provide a postoperative autotransfusion device that allows to interrupt the flow of blood toward the reinfusion container by activating a clamp for blocking the corresponding delivery tube.

Still another object of the present invention is to provide a postoperative autotransfusion device that is highly reliable, relatively simple to provide and at competitive costs.

This aim and these and other objects that will become better apparent hereinafter are achieved by a postoperative autotransfusion device, comprising a tank for collecting blood to be reinfused into a patient, according to the present invention that has the features set forth in claim 1.

Further characteristics and advantages of the present invention will become better apparent from the description of preferred but not exclusive embodiments of the device according to the invention, illustrated by way of non-limiting example in the accompanying drawings, wherein:
Figure 1 is a schematic view of a first embodiment of the device according to the present invention;
Figure 2 is a transverse sectional view of a second embodiment of the device according to the present invention in a first operating condition;
Figure 3 is a transverse sectional view of the device according to the present invention according to its second embodiment, in a second operating condition.

With reference to the figures, the device according to the present invention comprises a blood collection tank 1, which is provided, at an upper end, with at least a drained blood inlet 14 and, at its bottom, with at least a tube 2 for delivering blood to at least a collection container 3 that allows subsequently the reinfusion of the blood into the patient.

Alternatively, reference numeral 3 can represent the patient.

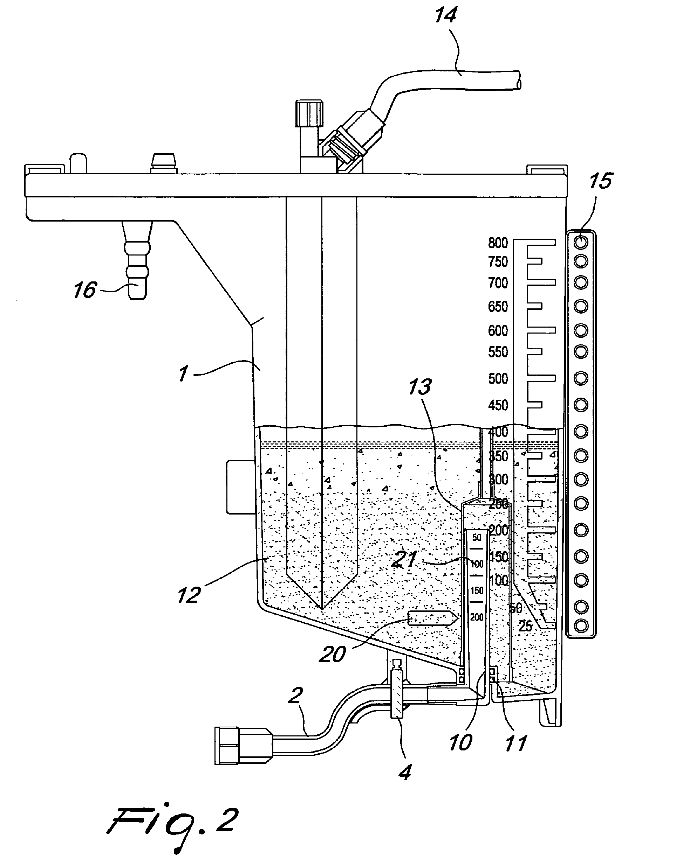

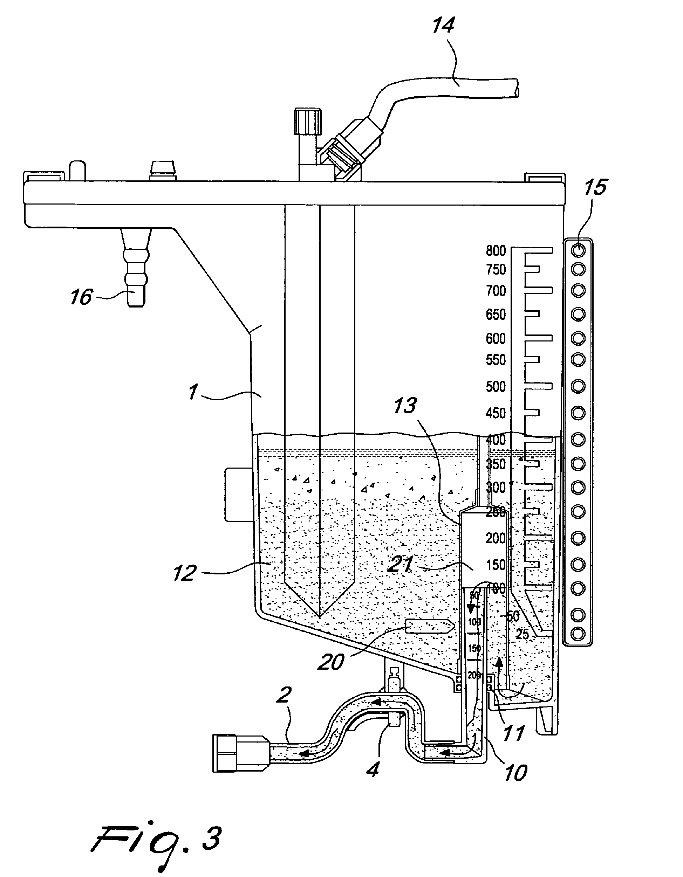

Conveniently, the collection tank 1 can be provided, as shown in Figures 2 and 3, so that its bottom blends substantially in a funnel-like shape toward the delivery tube 2, so as to facilitate the gathering of the red cells toward the bottom of the collection tank or container 1.

The collection tank 1 is also provided with at least a connector 16 for the application of a source of vacuum or atmospheric vent.

It should be noted that in the different embodiments, identical reference numerals designate identical elements.

The delivery tube is provided with clamping means advantageously comprising at least a clamp 4, which allows, when actuated manually or automatically, to clamp the tube 2 and thus prevent the flow of blood to the reinfusion container 3, or to leave the delivery tube 2 clear and thus allow the flow of blood to the reinfusion container 3.

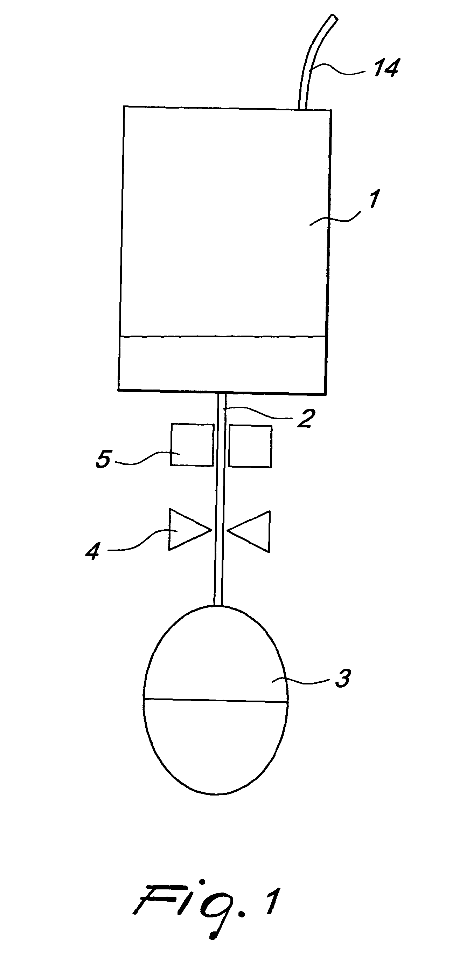

The peculiarity of the device according to the invention consists in that it has means adapted to determine the concentration of red cells in the blood collected in the collection tank 1. Such means, designated by the reference numeral 5, are conveniently constituted by sensor means that are adapted to be arranged, in the first embodiment shown in Figure 1, around the delivery tube 2, and are adapted to activate tube clamping means, i.e., the clamp 4, to interrupt the flow of blood to the reinfusion container 3 when the concentration of red cells in the blood that flows through the tube 2 is below a preset threshold.

Figures 2 and 3 are transverse sectional views of the second embodiment of the device according to the present invention, in which the means adapted to determine the concentration of red cells, designated by the reference numeral 5 in the first embodiment, are now designated by the reference numeral 15 and comprise means adapted to detect the light reflected by the container or tank 1 or means adapted to measure the light refracted by such container. As an alternative, means adapted to perform a spectral analysis of the reflected or refracted light can be provided.

Conveniently, the means 15 adapted to determine the concentration of red cells are provided with at least one visual indicator and preferably a plurality of visual indicators such as LEDs, adapted to light up in different colors depending on what is detected by the means 15, i.e., depending on whether the tank or container 1 is empty, or whether the means 15 have detected the presence of supernatant that is poor in red cells, or whether the means 15 have determined the presence of concentrated red cells at the concentration suitable for reinfusion into a patient.

The reference numeral 12 designates the blood that is present within the tank 1.

Conveniently, the collection tank 1 is provided with a supernatant separator partition, designated by the reference numeral 10, which can be movable manually (as a consequence of the determination made by the means 15 or on the basis of the visual control by the operator of the stratification within the collection tank) or automatically as a consequence of the determination performed by the means 15.

The supernatant separator partition is conveniently constituted by a tube with an open upper end, which is inserted in the container 1 at a bottom end thereof, and the seal between the tube and the walls of the container that define the inlet for the passage of the tube is entrusted to at least a gasket 11 of the O-ring type.

The tube 10 that constitutes the separator partition acts as a siphon, since it is accommodated in a bell-shaped container 13 that is open on the upper portion, which in turn is inserted in the collection tank 1 in which the blood 12 to be reinfused is present.

A graduated scale 21 is provided on the outer surface of the tube 10 of the supernatant separator partition and an indicator 20 that is adapted to point to the graduated scale is instead defined on the outer surface of the tank or container 1.

Figures 2 and 3 are views of two different operating conditions of the device according to the present invention: Figure 2 is a view of an operating condition in which the delivery tube 2 is clamped or closed, while Figure 3 is a view of the condition in which the tube 2 is opened and therefore the flow of blood from the collection tank 1 to the reinfusion container 3 is enabled.

Operation of the device according to the invention, in its two described and illustrated embodiments, is as follows.

First of all, the means 5, 15 adapted to determine the concentration of red cells determine the concentration of red cells and determine whether it is above a threshold value.

The means 5, 15 automatically keep open the clamp 4 that clamps the tube 2, as long as the concentration is the desired one, thus allowing the flow of concentrated red cells to the reinfusion container 3, i.e., it closes automatically the clamp 4 that clamps the tube 2 when the red cell concentration has dropped below the threshold level.

In the second embodiment, the means 5, 15 adapted to determine the concentration are arranged on the collection tank 1 and are adapted to actuate the opening or closure of the clamp 4 around the tube 2, so as to enable or disable the passage of blood from the collection tank 1 to the reinfusion container 3.

Conveniently, the supernatant separator partition 10 can move from a position as shown in Figure 2, with the clamp 4 in the condition for clamping the tube 2 and therefore blocking the flow, to a position as shown in Figure 3, in which the clamp 4 is open and therefore the tube 2 also is open and connects the collection tank 1 to the reinfusion container 3.

Therefore, the operator has to position manually the supernatant separator at the indicated level on the basis of visual inspection, by the operator, of the stratification within the tank, i.e., on the basis of the indication given by the means 15 adapted to determine the concentration of red cells, so as to have a correct threshold to discriminate between the red cells that can be sent to the reinfusion container 3, or blood components that must not be reinfused.

Positioning of the separator partition 10 can also occur automatically, as a consequence of the determination performed by the means 15 for determining the concentration of red cells in the blood 12.

In practice it has been found that the device according to the present invention fully achieves the intended aim and objects, since it allows to determine automatically the concentration of red cells in blood and therefore to allow or not the flow of the blood within the tube that leads from the collection tank to the reinfusion container.

The device thus conceived is susceptible of numerous modifications and variations, all of which are within the scope of the appended claims; all the details may further be replaced with other technically equivalent elements.

In practice, the materials used, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A postoperative autotransfusion device, comprising a tank (1) for collecting blood to be reinfused into a patient, connected by means of a tube (2) to a reinfusion container (3), and means (5, 15) adapted to determine the concentration of red cells in the blood (12) contained in the tank (1), in order to enable or not the flow of the blood to the reinfusion container (3), **characterized in that** it further comprises a partition (10) for separating supernatant/concentrated red cells which is inserted inside said collection tank (1), accommodated within a cavity, at an opening defined in the bottom said collection tank (1), and sealing means (11) that form a seal between said partition (10) and the walls that define the cavity within which said partition (10) is accommodated for insertion within said collection tank (1), said partition (10) being adapted to be moved as a consequence of a command sent by said means (15) adapted to determine the concentration of red cells in the blood (12) contained within said collection tank (1).

2. The device according to claim 1, **characterized in that** the means (5) adapted to determine the concentration of red cells in the blood contained in said collection tank are arranged on said tube (2).

3. The device according to claim 1, **characterized in that** said means (15) adapted to determine the concentration of red cells in the blood (12) contained within said collection tank (1) are arranged on said collection tank (1).

4. The device according to one or more of the preceding claims, **characterized in that** said means (5, 15) adapted to determine the concentration ofred cells in the blood (12) contained in said collection tank (1) are adapted to actuate clamping means (4) that are adapted to clamp or release the clamping of said tube (2) to block or allow the flow of said blood (12) from said collection tank (1) to said reinfusion container (3).

5. The device according to one or more of the preceding claims, **characterized in that** said means (15) adapted to determine the concentration of red cells in the blood (12) contained in said collection tank (1) are adapted to measure the light reflected by the blood (12) contained in said collection tank (1).

6. The device according to one or more of the preceding claims, **characterized in that** said means (15) adapted to determine the concentration of red cells in the blood (12) contained in said collection tank (1) are adapted to measure the light refracted by the blood (12) contained in said collection tank (1).

7. The device according to one or more of the preceding claims, **characterized in that** said means (15) adapted to determine the concentration of red cells in the blood (12) contained in said collection tank (1) are adapted to perform a spectral analysis of the light reflected or refracted by the blood (12) contained in said collection tank (1).

8. The device according to one or more of the preceding claims, **characterized in that** said means (15) adapted to determine the concentration of red cells in the blood (12) contained in said collection tank (1) comprise at least an indicator that is, adapted to indicate lack of blood, presence of blood poor in red cells; and presence of concentrated red cells.

9. The device according to any of the preceding claims, **characterized in that** said supernatant separator partition (10) is constituted by a tube with an open upper end, which is inserted in the collection container (1) at a bottom end thereof, said sealing means that form the seal between said tube (10) and the walls of the container that define the inlet for the passage of the tube (10) being constituted by at least one gasket (11).

10. The device according to claim 9, **characterized in that** said tube (10) that constitutes the separator partition acts as a siphon, being accommodated within a bell-shaped container (13) that is open in an upward region, said bell-shaped container (13) being in turn inserted in said collection tank (1) in which the blood (12) to be reinfused is present.

11. The device according to claim 10, **characterized in that** it comprises a graduated scale (21) that is defined on the outer surface of said tube (10) of the supernatant separator and an indicator (20) that is adapted to point to said graduated scale (21) and is defined on the outer surface of the collection tank (1).

## Patentansprüche

1. Vorrichtung zur postoperativen Autotransfusion mit einem Behälter (1) zum Auffangen des Blutes, das dem Patienten wieder zugeführt wird, der mittels eines Rohres (2) mit einem Reinfusionsbehälter (3) verbunden ist, und Mitteln (5, 15) zur Bestimmung der Konzentration roter Blutkörperchen im Blut (12), das in dem Behälters (1) enthalten ist, um den Blutfluss zu dem Reinfusionsbehälter (3) zu ermöglicher oder nicht, **dadurch gekennzeichnet, dass** sie weiterhin eine Trennwand (10) besitzt zum Abtrennen überstehender/konzentrierter roter Blutkörperchen, die in den Behälters (1) eingefügt ist und innerhalb eines Hohlraums aufgenommen wird, an einer Öffnung, die sich in dem Boden des Behälters (1) befindet, und Abdichtelemente (11), die eine Abdichtung zwischen der Trennwand (10) und den Wänden bilden, welche den Hohlraum darstellen, innerhalb dessen die Trennwand (10) für den Einschub innerhalb des Behälters (1) aufgenommen ist, wobei die Trennwand (10) aufgrund eines Befehls, der von den Mitteln (15) gesendet wird, welche die Konzentration von roten Blutkörperchen in dem Blut (12) bestimmen, das in dem Behälter (1) enthalten ist, verschoben werden kann.

2. Vorrichtung nach Anspruch 1, **dadurch gekennzeichnet, dass** sich die Mittel (5) zur Bestimmung der Konzentration roter Blutkörperchen im Blut, das in dem Behälter enthalten ist, an dem Rohr (2) befinden.

3. Vorrichtung nach Anspruch 1, **dadurch gekennzeichnet, dass** die Mittel (15) zur Bestimmung der Konzentration von roten Blutkörperchen in dem Blut (12), die sich innerhalb des Behälters (1) befinden, an dem Behälter (1) angeordnet sind.

4. Vorrichtung nach einem oder mehreren der vorhergehenden Ansprüche, **dadurch gekennzeichnet, dass** die Mittel (5, 15) zur Bestimmung der roten Blutkörperchen in dem Blut (12), das in dem Behälter (1) entfalten ist, Klemmelemente (4) betätigen, die die Einspannung des Rohres (2) festhalten oder lösen, um den Blutfluss (12) von dem Behälter (1) zu dem Reinfusionsbehälter (3) zu blockieren oder zuzulassen.

5. Vorrichtung nach einem oder mehreren der vorhergehenden Ansprüche, **dadurch gekennzeichnet dass** die Mittel (15) zur Bestimmung der Konzentration der roten Blutkörperchen in dem Blut (12), das in dem Behälter (1) entfalten ist, das Licht messen, das von dem Blut (12), welches in dem Behälter (1) enthalten ist, reflektiert wird.

6. Vorrichtung nach einem oder mehreren der vorhergehenden Ansprüche, **dadurch gekennzeichnet, dass** die Mittel (15) zur Bestimmung der Konzentration der roten Blutkörperchen in dem Blut (12), das in dem Behälter (1) enthalten ist, das Licht messen, das von dem Blut (12), welches in dem Behälter (1) enthalten ist, gebrochen wird.

7. Vorrichtung nach einem oder mehreren der vorhergehenden Ansprüche, **dadurch gekennzeichnet, dass** die Mittel (15) zur Bestimmung der Konzentration der roten Blutkörperchen in dem Blut (12), das in dem Behälter (1) enthalten ist, eine Spektralanalyse des Lichtes, das von dem Blut (12), das in dem Behälters (1) entfalten ist, reflektiert oder gebrochen wird, durchführen können.

8. Vorrichtung nach einem oder mehreren der vorhergehenden Anspruche, **dadurch gekennzeichnet, dass** die Mittel (15) zur Bestimmung der Konzentration der roten Blutkörperchen in dem Blut (12), das in dem Behälter (1) enthalten ist, mindestens einen Indikator enthalten, der das Fehlen von Blut, das Vorhandensein von Blut arm an roten Blutkörperchen und das Vorhandensein konzentrierter roter Blutkörperchen anzeige.

9. Vorrichtung nach einem der vorhergehenden Ansprüche, **dadurch gekennzeichnet, dass** die überstehende Trennwand (10) aus einem Rohr mit einem offenen oberen Ende besteht, das in den Behälter (1) an dessen unterem Ende eingeführt wird, wobei die Abdichtelemente, welche die Abdichtung zwischen dem Rohr (10) und den Wänden des Behälters bilden, die den Einlass für den Durchgang des Rohres (10) darstellen, aus mindestens einer Dichtung (11) bestehen.

10. Vorrichtung nach Anspruch 9, **dadurch gekennzeichnet, dass** das Rohr (10), das die Trennwand bildet, wie ein Siphon wirkt, der innerhalb eines glockenförmigen Behälters (13) aufgenommen ist, welcher nach oben geöffnet ist, wobei der glockenförmig Behälters (13) seinerseits in den Behälter (1) eingeschoben ist, in dem sich das Blut (12) befindet, das dem Patienten wieder zugeführt wird.

11. Vorrichtung nach Anspruch 10, **dadurch gekennzeichnet, dass** sie eine Gradskala (21) aufweist, die sich an der Außenseite des Rohres (10) der überstehenden Trennwand befindet, und einen Zeiger, der auf die Gradskala (21) zeigt, und der sich an der Außenseite des Behälters (1) befindet.

## Revendications

1. Dispositif d'autotransfusion post-opératoire, comprenant un réservoir (1) pour collecter le sang destiné à être retransfusé à un patient, raccordé au moyen d'un tube (2) à un contenant (3) de retransfusion, et des moyens (5, 15) aptes à déterminer la concentration en érythrocytes dans le sang (12) contenu dans le réservoir (1) de manière à permettre ou non l'écoulement du sang vers le contenant (3) de retransfusion, ***caractérisé en ce qu*'**il comprend en outre une cloison (10) pour une séparation surnageant/érythrocytes concentrés, cloison qui est insérée à l'intérieur dudit réservoir (1) de collecte, reçue dans une cavité, au niveau d'une ouverture ménagée dans le fond dudit réservoir (1) de collecte, et des moyens d'étanchéité (11) qui forment un joint étanche entre ladite cloison (10) et les parois qui définissent la cavité à l'intérieur de ladite toison (10) sont pourvus pour l'insertion à l'intérieur dudit réservoir (1) de collecte, ladite toison (10) étant apte a être déplacée en réponse à une commande envoyée par lesdits moyens (15) aptes à déterminer la concentration en érythrocytes dans le sang (12) contenu dans ledit réservoir (1) de collecte.

2. Dispositif selon la revendication 1, ***caractérisé en ce que*** les moyens (5) aptes à déterminer la concentration en érythrocytes dans le sang contenu dans ledit réservoir de collecte sont placés sur ledit tube (2).

3. Dispositif selon la revendication 1, ***caractérisé en ce que*** lesdits moyens (15) aptes à déterminer la concentration en érythrocytes dans le sang (12) contenu dans ledit réservoir (1) de collecte sont placés sur ledit réservoir (1) de collecte.

4. Dispositif selon l'une ou plusieurs des revendications précédentes, ***caractérisé en ce que*** lesdits moyens (5, 15) aptes à déterminer la concentration en érythrocytes dans le sang (12) contenu dans ledit réservoir (1) de collecte sont aptes à déclencher des moyens de serrage (4) qui sont aptes à serrer ou à relâcher le serrage dudit tube (2) pour bloquer ou permettre l'écoulement dudit sang (12) dudit réservoir (1) de collecte audit contenant (3) de retransfusion.

5. Dispositif selon l'une ou plusieurs des revendications précédentes, ***caractérisé en ce que*** lesdits moyens (15) aptes à déterminer la concentration en érythrocytes dans le sang (12) contenu dans ledit réservoir (1) de collecte sont aptes à mesurer la lumière réfléchie par le sang (12) contenu dans ledit réservoir (1) de collecte.

6. Dispositif selon l'une ou plusieurs des revendications précédentes, ***caractérisé en ce que*** lesdits moyens (15) aptes à déterminer la concentration en érythrocytes dans le sang (12) contenu dans ledit réservoir (1) de collecte sont aptes à mesurer la lumière réfractée par le sang (12) contenu dans ledit réservoir (1) de collecte.

7. Dispositif selon l'une ou plusieurs des revendications précédentes, ***caractérisé en ce* que** lesdits moyens (15) aptes à déterminer la concentration en érythrocytes dans le sang (12) contenu dans ledit réservoir (1) de collecte sont aptes à réaliser une analyse spectrale de la lumière réfléchie ou réfractée par le sang (12) contenu dans ledit réservoir (1) de collecte.

8. Dispositif selon l'une ou plusieurs des revendications *précédentes, **caractérisé en ce que*** lesdits moyens (15) aptes à déterminer la concentration en érythrocytes dans le sang (12) contenu dans ledit réservoir (1) de collecte comprennent au moins un indicateur qui est apte à indiquer l'absence de sang, la présence de sang pauvre en érythrocytes, et la présence d'érythrocytes concentrés.

9. Dispositif selon l'une quelconque des revendications précédentes, ***caractérisé en ce* que** ladite cloison (10) de séparation du surnageant est constituée par un tube avec une extrémité supérieure ouverte, qui est inséré dans le réservoir (1) de collecte à une extrémité inférieure de celui-ci, lesdits moyens d'étanchéité qui forment un joint étanche entre ledit tube (10) et les parois du contenant qui définissent l'entrée pour le passage du tube (10) étant constitués par au moins une bague d'étanchéité (11).

10. Dispositif selon la revendication 9, ***caractérisé en ce que*** ledit tube (10) qui constitue la cloison de séparation sert de siphon, étant reçu dans un récipient en forme de cloche (13) qui est ouvert dans une zone située sur le haut, ledit récipient en forme de cloche (13) étant à son tour inséré dans ledit réservoir (1) de collecte dans lequel est présent le sang (12) à retransfuser.

11. Dispositif selon la revendication 10, ***caractérisé en ce qu'**il* comprend une règle graduée (21) qui est définie sur la surface extérieure dudit tube (10) du séparateur du surnageant et un indicateur (20) qui est apte à pointer vers ladite règle graduée (21) et est défini sur la surface extérieure du réservoir (1) de collecte.
